# EUROPEAN PATENT SPECIFICATION

(11) **EP 4 188 406 B1**
(45) Date of publication and mention of the grant of the patent: **26.06.2024**
(21) Application number: 21758077.8
(22) Date of filing: 02.08.2021
(51) Int. Cl.: A61K 36/185, A61K 36/28, A61K 36/324, A61K 9/14, A23L 33/105, A23L 33/175, A61K 36/328

(54) **COMPOSITION BASED ON MYRRH**
ZUSAMMENSETZUNG AUF MYRRH-BASIS
COMPOSITION À BASE DE MYRRHE

(30) Priority: 03.08.2020 IT 202000018970
(43) Date of publication of application: 07.06.2023
(73) Proprietor: Asoltech Srl, 34018 San Dorligo della Valle (TS) (IT)
(72) Inventor: CANAL, Tiziana, 34018 San Dorligo della Valle (TS) (IT); FORTUNA, Fulvio, 34018 San Dorligo della Valle (TS) (IT); MENCINI, Elena, 34018 San Dorligo della Valle (TS) (IT); CATANESE, Maurizio Danilo, 20090 Trezzano sul Naviglio (MI) (IT)
(74) Representative: Tagliafico, Giulia
(86) International application number: PCT/EP2021/071549
(87) International publication number: WO 2022/029067

(56) References cited:
- WO-A1-98/42188
- WO-A1-2015/198346
- DATABASE GNPD [Online] MINTEL; 18 April 2018 (2018-04-18), MINTEL: "Hair Growth Lotion", XP55632931, Database accession no. 5598249
- DATABASE GNPD [Online] MINTEL; 18 April 2018 (2018-04-18), anonymous: "Hair Growth Lotion", XP55632931, Database accession no. 5598249

## Description

### FILED OF THE INVENTION

The present invention relates to a composition comprising a dry extract of *Commiphora myrrho,* fructose or mannitol and sucrose esters, optionally with aminoacids, obtained by dry co-grinding of its components, optionally comprising a further ingredient, such as for example a dry extract of *Boswellia serrata, Pelargonium sidoides, Echinacea purpurea* or propolis, provided that said composition obtained by co-grinding does not contain polymeric excipients

### BACKGROUND OF THE INVENTION

Myrrh is an aromatic gum, extracted from some trees and shrubs belonging to the Commiphora genus, of the Burseraceae family.

Recent studies have shown that myrrh is made from 3 to 8% of essential oils, from 25 to 40% of resins soluble in alcohol and from 30 to 60% of gums soluble in water [Hamm S., Bleton J., Tchapla A. Micro-extraction in solid phase in headspace for screening for the presence of resins in Egyptian archaeological samples, J. Sep. Sci. 2004; 27: 235-243]. The chemical components of the essential oils comprise monoterpenes, sesquiterpenes and small aromatic molecular compounds. The chemical compounds of the resins comprise diterpenoids, triterpenoids, steroids and lignans.

The properties of myrrh (*Commiphora myrrho* extract) known since ancient times particularly depend on the presence of bioactive sesquiterpenes with a furanodienic structure.

The main characteristic of these compounds is the poor solubility in water; in fact, in order to obtain extracts titrated in furanodienes , the resin of myrrh is usually treated with non polar or slightly polar solvents, such as for example hexane, ether, ethyl alcohol.

Myrrh is often also used in combination with other extracts, for example in combination with an extract of *Boswellia serrata* [see, for example, Letters in Applied Microbiology 54, 352-358, 2012, The additive and synergistic antimicrobial effects of select frankincense and myrrh oils - a combination from the pharaonic pharmacopoeia. S. de Rapper et al./ Front. Pharmacol. 9:686, 2018 Googling the Guggul (*Commiphora* and *Boswellia*) for Prevention of Chronic Diseases. Kunnumakkara AB,et al./ Molecules 2019, 24, 3076; Seeing the Unseen of the Combination of Two Natural Resins, Frankincense and Myrrh: Changes in Chemical Constituents and Pharmacological Activities. Bo Cao, et al.].

Myrrh is also described in combination with propolis [see for example, Biofouling. 2019 Aug;35(7):796-804, Effect of commercial herbal toothpastes and mouth rinses on the prevention of enamel demineralization using a microcosm biofilm model; Braga AS, et al. / Br J Biomed Sci. 2006;63(4):171-3, Combined use of honey, bee propolis and myrrh in healing a deep, infected wound in a patient with diabetes mellitus. Lotfy M, et al.].

Myrrh combinations with echinacea are cited, for example in "A Clinical Guide to Blending Liquid Herbs: Herbal Formulations for the Individual Patient", 1e Hardcover - 25 Sept. 2003, Kerry Bone, Churchill Livingstone Ed. / An Herbalist's Guide to Growing & Using Echinacea, A Storey Country Wisdom Bulletin by Brown, Kathleen (1999), or are offered to the public, as, for example, in the product Swanson Full Spectrum Echinacea & Myrrh Gum, 60 capsules.

Compositions consisting of mixed combinations of these ingredients with other ingredients are known and described in published patent applications, such as for example the following:
1) KR102057790 reports compositions comprising antiviral agents including boswellia, myrrh, propolis, olive leaves extract, sacha inchi oil, methylsulfonylmethane (MSM) in liquid or extract form;
2) KR20190101318 indicates an antifungal composition comprising *Coix lacryma-jobi* var.mayuen, xylytol, vitamin C, manuka honey, *Boswellic serrata,* myrrh and propolis in powder, in liquid form or extract;
3) KR20190036628 mentions a cosmetic composition containing myrrh and gold, further mixed with one or more functional materials from liquid extracts of propolis and ginseng;
4) WO2020105070 relates to a food supplement comprising the following active ingredients: Bugloxoid seed oil, pineapple, fluid extract of boswellia, fluid extract of myrrh;
5) CN104489279 mentions a composition based on *Houttuynia cordata,* brook anemone root, *Fructus liquidambaris,* common peony root, scrofularia root, boswellia, myrrh, echinacea, astragalus and licorice;
6) NZ528834 indicates a formulation for oral health comprising: sodium bicarbonate plus a mixture of natural ingredients with comprise echinacea, myrrh, chamomile, rhodania and sage; and
7) WO2019116218 reports an association of plant extracts, which includes myrrh and ginger.

The study of said documents relating the combination of myrrh and other extracts shows that these are obtained by simply mixing the components, whether in liquid or powder form, optionally with other excipients, but no document deals with the problem of the poor solubility of myrrh which, as it is well known, can affect the absorption and therefore the actual effectiveness of these substances or combinations [see for example Savjani KT, Gajjar AK, Savjani JK. Drug solubility: importance and enhancement techniques. ISRN Pharm. 2012;2012:195727].

The same article mentions techniques commonly used for the improvement of solubility.

Almost all (solid dispersions, melting, solvent evaporation, extrusion, coprecipitation, high pressure homogenization, freeze- and spray-drying, formation of inclusion complexes) require the presence of at least one solvent and/or of a polymeric carrier or capable to form inclusion complexes or the combination of both.

The use of solvents poses the problem of possible undesired residues in the product and of their recovery and disposal, besides requiring processes in more steps and therefore longer and more expensive.

The use of carriers able to form inclusion complexes has the disadvantage of requiring precise stoichiometric ratios, thus limiting the formulation flexibility; furthermore not always the hydrophobic cavity of the carrier (for example cyclodextrins) is large enough to accommodate the host molecule.

Furthermore, as well as in the case of the polymeric carriers, it is necessary to find a common solvent, or, in the case of process requiring the fusion, thermally compatible materials to avoid degradation.

Solubility is often intrinsically related to the size of the particles; the conventional tecniques to reduce the particle size and increase the solubility, such as comminution or micronization, are based on mechanical stresses and the mechanical forces involved often impart physical stresses to the product, which can induce degradation. In addition, the thermal stress, which can occur, can be a problem during the processing of thermosensitive or unstable active ingredients.

Myrrh, boswellia and propolis, moreover, have a particularly bitter and pungent taste, a characteristic present, even if to a lesser extent, also in echinacea and pelargonium.

The technical problem to be solved in relation to compositions containing myrrh is therefore, in addition to the increase of its solubility, the masking of this bad taste.

A known technique used to increase the solubility, in particular used with coenzyme Q10, provides for the dry grinding of coenzyme Q10 with maltodextrin and sucresters (WO2013080028) or hydroxypropylcellulose and sucresters (WO2016074800). Such technique has the advantage of not requiring solvents and maintaining low process temperatures. To obtain the desired effect, however, it is necessary to use polymeric carrier.

### DESCRIPTION OF THE INVENTION

The main purpose of the present invention is that of increasing the solubility and masking the taste of a composition comprising a dry extract of *Commiphora myrrho,* optionally in combination with dry extracts selected from: *Boswellia serrata, Pelargonium sidoides, Echinacea purpurea* and propolis.

Using the conditions and the material described in the above mentioned technique (thus dry co-grinding with maltodextrin and sucresters or hydroxypropylcellulose and sucresters) it was not possible to obtain a masking effect of the flavor regarded as suitable; the addition of compounds such as fructose or mannitol still did not allow to obtain satisfactory results in masking the bitter / pungent taste.

We have now surprisingly found that by dry co-grinding *Commiphora myrrho* or one of its binary combinations with another extract selected from *Boswellia serrata, Pelargonium sidoides, Echinacea purpurea* or with propolis, in the presence of only fructose or mannitol and sucresters, optionally with amino acids, in addition to improving the flavor, an increase of the solubility of the myrrh and its combinations with other extracts or ingredients is achieved, even in the absence of carrier or polymeric excipients.

It is therefore object of the present invention a composition comprising a dry extract of *Commiphora myrrho,* fructose or mannitol and sucresters, optionaly with amino acids, obtained by dry co-grinding of its components, optionally comprising a further ingredient, such as for example a dry extract of *Boswellia serrata, Pelargonium sidoides, Echinacea purpurea* or propolis, provided that said composition obtained by co-grinding does not contain polymeric excipients.

By "dry co-grinding" it is meant a process, wherein a mixture of the substances in powder form are ground dry, that is without the addition of solvents or their mixtures, in a suitable equipment, such as for example a ball, hammer, planetary or vibrational mill, whether fixed body or rotating body. The powders can be premixed or mix during the co-grinding itself. At the end of the process of dry co-grinding, the powder composition obtained is discharged from the mill and optionally sieved, for example to eliminate any aggregates.

According to the present invention the word "myrrh" it is meant to refer to a dry extract of *Commiphora myrrha*; with the word "boswellia" it is meant to refer to a dry extract of *Boswellia serrata*; with the word "pelargonium" it is mean to refer to a dry extract of di *Pelargonium sidoides*; and with the word "echinacea" it is intended to refer to a dry extract of *Echinacea purpurea.*
With the word "propolis" it is intended to refer to a dry extract of propolis.

The "sucresters" are compositions which are obtained from the esterification of fatty acids or from transesterification of the mehyl esters of fatty acids with carbohydrates, usually sucrose and other polysaccharides. They are also referred to as sugar polyesters (o SPE from the English Sugar Poly Esters) or saccharose esters of fatty acids. Examples of sucresters are: sucrose monopalmitate, sucrose monostearate, sucrose dipalmitate, sucrose distearate, alkylated sucrose or mixtures of two or more of them.

Aminoacids which are optionally used in the co-ground compositions of the present invention are for example: the 20 standard amino acids (alanine, phenylalanine, glycine, isoleucine, leucine, methionine, proline, tryptophan, valine, asparagine, glutamine, serine, threonine, cysteine, tyrosine, aspartic acid, glutamic acid, arginine, histidine and lysine). As non-limiting example, preferred amino acids are: arginine, glycine, serine and glutamic acid.

The compositions of the present invention can be offered for sale to the final consumer as such or they can be used for the production of food supplements, food for special medical purposes or medical devices.

It is a further object of the present invention a food supplement comprising the composition of the present invention in one of the previously illustrated forms.

The term "food supplement (FS)" means a food product intended to supplement the common diet and which constitutes a concentrated source of nutrients, such as vitamins and minerals, or of other substances having a nutritional or physiological effect, in pre-dosed forms (see also Directive 2002/46 / EC of 10 June 2002 and Italian Legislative Decree 21 May 2004 no. 169 art. 2).

Also an object of the present invention is a food for special medical purposes (FSMP) containing the composition of the present invention in one of the forms previously illustrated.

The expression "food for special medical purposes (FSMP)" is understood as defined in the EU Regulation (No. 609/2013), as well as in the Guidelines issued by the EFSA (European Food Safety Authority) entitled "Scientific and technical guidance on food for special medical purposes in the context of Article 3 of Regulation (EU) No 609/2013" - EFSA Journal 2015; 13(11):430. In particular, this regulatory definition is divided into three points:
1) food specially processed or formulated and intended for the dietary management of patients, including infants, to be used under medical supervision;
2) intended for the exclusive or partial feeding of patients with a limited, impaired or disturbed capacity to take, digest, absorb, metabolize or excrete ordinary foods or certain nutrients contained therein, or metabolites, or with other medically-determined nutrient requirements;
3) whose dietary management cannot be achieved by modification of the normal diet alone . To be proposed and classified as an FSMP, a product must meet the three points of the previous definition.

For the purposes of the distinction with FS, it should also be noted that, while the latter products comply with the regulatory definition of Directive 2002/46 / EC, even if they only perform beneficial "physiological" effects for the supply of substances other than nutrients without any participation in the constitution of the food ration (for example products based on plant extracts), FSMPs must necessarily have a "nutritional" role, as constituents of a food ration aimed at satisfying the nutritional needs of patients in specific conditions of nutritional vulnerability.

It is also an object of the present invention a medical device comprising the composition of the present invention in one of the forms previously illustrated.

The expression "medical device" means a product, used alone or in combination intended by the manufacturer to be used on humans for the purpose of:
- diagnosis, prevention, control, therapy or alleviation of a disease;
- diagnosis, control, therapy, alleviation or compensation of a wound or a handicap;
- study, replacement or modification of the anatomy or of a physiological process
whose main action intended in or on the human body is not achieved by pharmacological or immunological means or by metabolism, but whose function can be assisted by these means (see Directive 93/42 / EEC of 14 June 1993 concerning medical devices and its subsequent amendments).

As regards the excipients and / or diluents that can be used in the compositions according to the invention to be placed on the market, please refer to what is widely reported in the literature and well known to experts in pharmaceutical technologies.

By pre-dosed forms we mean the usual pharmaceutical forms or formulations [see for example Principles of Pharmaceutical Technology, Paolo Colombo, Marco Adami, et al, CEA, 2015] made by mixing one or more substances with a nutritional or physiological effect with appropriate inert excipients to allow the technical realization of the finished product.

As non limiting example, pharmaceutical forms classified according to their physical form are the following:
- solid pharmaceutical forms (tablets, pills, capsules, powders, granules or sachets);
- semi-solid pharmaceutical forms (gels, creams, ointments, lubricants or pastes);
- liquid pharmaceutical forms (syrups, ampoules, drops, eye drops or sprays);
- gaseous pharmaceutical forms (aerosols);

Depending on the route of administration, pharmaceutical forms can be divided into:
- oral (solutions, emulsions, suspensions, powders, granules, capsules, tablets or sprays);
- topic (solutions, emulsions, cream, ointments, lotions or powders);
- parenteral (emulsion, solutions, suspensions, implants);
- rectal (solutions, emulsions, suppositories, ointments, powders);
- ophthalmic, vaginal, inhalatory, etc...

Considering the release time of the active components, they can also be classified into conventional, or immediate release, or modified release, i.e. prolonged, delayed or repeated release.

Finally, single-dose and multidose pharmaceutical forms, which must be dosed by the patient using special measuring cups or droppers, can be mentioned.

In the production of food supplements, the compositions of the present invention can also be associated with, assembled or mixed with one or more substances, such as for a non-limiting example, those belonging to the groups listed below:
- vitamins;
- minerals or metals;
- substances with a nutritional or physiological effect;
- vegetable substances and / or preparations or extracts; and
- prebiotics and probiotics.

The expression "associate", "assemble" or "mix" means that to the compositions of the present invention are added by the formulator skilled in the art, wishing to obtain a particular nutritional or physiological effect, one or more of the substances belonging to the groups listed above, so that the final formulation, to all effects, includes one of the compositions of the present invention *per se* and one or more substances belonging to the groups listed above.

Another embodiment of the present invention provides that the dry co-ground composition of the invention is added to a liquid composition for electronic cigarettes (*e-cig*), for example in order to protect and strengthen the immune system and/or to prevent possible bacterial and viral infections.

One embodiment provides a pharmaceutical form, suitably selected from those listed above, which comprises a composition according to the present description consisting of myrrh / pelargonium / fructose/sucresters.

This embodiment can assist the body's ability to respond to aggressive factors and the attack of infectious agents thanks to the antibacterial action of the myrrh component [see, for example, "Evaluation of the antibacterial efficacy of Azadirachta Indica, Commiphora Myrrho, Glycyrrhiza Glabra against Enterococcus Faecalis using Real Time PCR "- An and S. et al. - Open Dent J. 2016; 10: 160-165*]* and to the immunostimulating and antiviral/antibacterial component of the pelargonium component [see, for example, "Antibacterial Spectrum of Umckaloabo (Pelargonium Sidoides) On Upper Airway Infection Agents ", Hakan Uslu, Eur J Gen Med 2009; 6 (4): 245-248;" Antimicrobial, Antiviral and Immunomodulatory Activity Studies of Pelargonium sidoides (EPs® 7630) in the Context of Health Promotion ", Herbert Kolodziej, Pharmaceuticals 2011, 4, 1295-1314].

A further embodiment provides a pharmaceutical form, suitably selected from those listed above, which comprises a composition according to the present description consisting of myrrh/boswellia/mannitol sucresters/arginine. This embodiment can assist the body's ability to respond to inflammatory factors and related pains thanks to the analgesic action of the myrrh component [see for example, Massimo E. Maffei, The Nutritional Supplement, 2017, 20 (3), p. 48-55 e A Pilot Study on Bioactive Constituents and Analgesic Effects of MyrLiq^{®} a Commiphora myrrha Extract with a High Furanodiene Content. Germano, A. (2017) BioMed Research International] and to the anti-inflammatory component of the boswellia component [see, for example, "A pilot, randomized, double-blind, placebo-controlled trial to assess the safety and efficacy of a novel Boswellia serrata extract in the management of osteoarthritis of the knee", Phytotherapy Research. Majeed, M. (2019) 33:1457-1468].

A further embodiment provides a pharmaceutical form, suitably selected from those listed above, which comprises a composition according to the present description consisting of myrrh/echinacea/fructose/sucresters.

This embodiment can assist in increasing immune defenses and preventing flu and para-flu states thanks to the antibacterial action of the myrrh component [see for example Shaik, Janbee & K, Vishakha & D, Ramyasree. (2015), "Evaluation of antibacterial activity of Commiphora myrrha against antibiotic resistant clinical pathogens", Indian Journal of Pharmaceutical and Biological Research] and to the immunostimulating component of the echinacea component [see, for example, , "Safety and Efficacy Profile of Echinacea purpurea to Prevent Common Cold Episodes: A Randomized, Double-Blind, Placebo-Controlled Trial", M. Jawad, Evidence-Based Complementary and Alternative Medicine, Volume 2012, Article ID 841315, p.7].

A further embodiment provides a pharmaceutical form, suitably selected from those listed above, which comprises a composition according to the present description consisting of myrrh/propolis/mannitol/sucresters. This embodiment can assist the body's ability to respond to aggressive factors and the attack of infectious agents thanks to the antibacterial action of the myrrh component [see, for example, "Antibacterial terpenes from the oleo-resin of Commiphora molmol (Engl.), M. Mukhlesur Rahman, Phytother. Res. 22, 1356-1360 (2008)] and to the antiviral / antibacterial one [see, for example, Shimizu T, Hino A, Tsutsumi A, Park YK, Watanabe W, Kurokawa M. Anti-influenza virus activity of propolis in vitro and its efficacy against influenza infection in mice. Antivir Chem Chemother. 2008;19(1):7-13] of the propolis component.

According to an aspect of the invention, each dry extract is present in the dry co-ground composition of the present invention in a ratio comprised between 5 and 50% by weight, preferably between 10 and 30% in weight; the fructose or mannitol are present in the composition in a ratio comprised between 20 and 80% in weight, preferably between 40 and 60% in peso; the sucresters, optionally with aminoacids, are present in the composition in a ratio comprised between 5 and 40% in weight, preferably between 10 and 30% in weight.

The following examples illustrate the invention without limiting it and refer to the following Figures and Tables. Examples are also reported relating to co-ground of myrrh alone with fructose or mannitol to demonstrate that the solubility of myrrh alone is also increased.

### DESCRIPTION OF THE FIGURES

Figure 1: shows the UV spectra of the physical mixtures of the components and of the respective co-ground, in order to highlight the comparison between the respective solubilities, in this case relative to the compositions of Examples 1 and 2: MIX FIS M1 vs. EXAMPLE 1 and MIX FIS M2 vs. EXAMPLE 2.

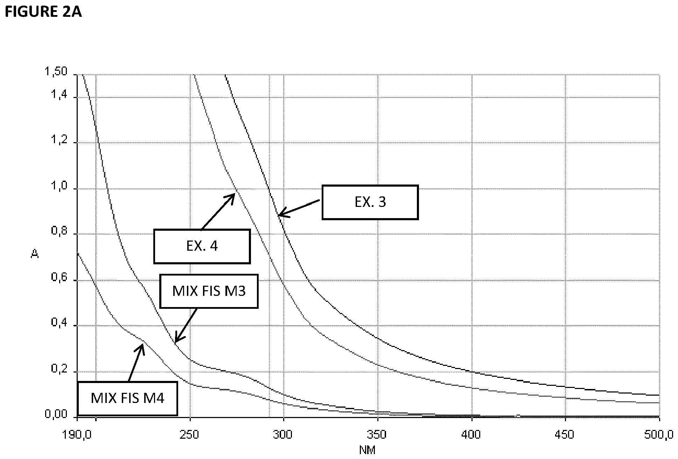

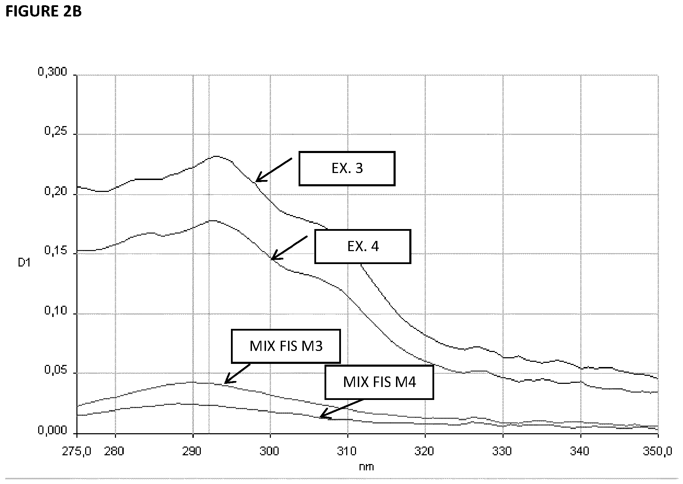

Figure 2: shows the UV spectra of the physical mixtures of the components of of the respective co-ground, in order to highlight the comparison between the respective solubilities, in this case relative to the compositions of Examples 3 and 4: MIX FIS M3 vs. EXAMPLE 3 and MIX FIS M4 vs. EXAMPLE 4. In this case it was not easy to determine at which wavelength to perform the absorbance readings. We therefore resorted to derivative processing before the spectrum to transform the inflection point into a peak. This method made it possible to identify the wavelength of 292 nm as significant. The first derivative spectrum is shown in Figure 2B. This wavelength (292 nm) was then used to determine the absorbance in the spectra of Figure 2A.
Figure 3: shows the UV spectra of the physical mixtures and of the respective co-ground, in order to highlight the comparison between the respective solubilities, in this case relative to the compositions of Examples 5 and 6: MIX FIS M5 vs. EXAMPLE 5 and MIX FIS M6 vs. EXAMPLE 6.
Figure 4: shows the UV spectra of the physical mixtures of the components and of the respective co-ground, in order to highlight the comparison between the respective solubilities, in this case relative to the compositions of Examples 7 and 8: MIX FIS M7 vs. EXAMPLE 7 and MIX FIS M8 vs. EXAMPLE 8.
Figure 5: shows the UV spectrum of myrrh, of the physical mixture of the components of Example A and of the co-ground, in order to highlight the comparison between the respective solubilities, in this case between the solubility of the myrrh and the co-ground of the Examples A and B. The solubility of the physical mixture relating to Example A (MIX FIS MA) is also reported.

### EXAMPLES

The extracts listed in the following examples have been found from usual suppliers/retailers [such as by way of non-exhaustive example: EPO Extracts of Officinal Plants Srl, Fizzonasco di Pieve Emanuele (MI); Nutraceutica, Monterenzio (BO); CARLO SESSA S.P.A., Sesto San Giovanni (Ml); A.C.E.F. Spa. Azienda Chimica e Farmaceutica Fiorenzuola D'Arda (PC)].

Also the excipients were found from usual suppliers/retailers [such as by way of non-exhaustive example: Prodotti Gianni Srl, Milano; Rader S.P.A. Altavilla Vicentina (VI); Giusto Faravelli SpA, Milano )].

In the case of botanical extracts, the parts of the plants used are those authorized by Annex 1 to the Italian Health Ministry Decree of 10 August 2018 as amended by the Executive Decree of 26 July 2019.

### EXAMPLE 1

### Composition myrrh/pelargonium/fructose/sucresters in a ratio in ratio w/w 10/20/40/30

For the preparation of the composition of this example, 30 g of a mixture in the ratio w/w 10/20/40/30 of myrrh (3 g)/pelargonium (6 g)/fructose (12 g)/sucresters (9 g), obtained using a rotating body mixer, were loaded into a jar of a planetary mill and subjected to co-grinding for 30 minutes at a speed of 200 rpm. At the end of the process, the product in a fine powder form was unloaded and sieved. A co-ground myrrh/pelargonium/fructose/sucresters in a ratio w/w 10/20/40/30 was obtained titrated in myrrh at 10% by weight and in pelargonium at 20% by weight.

### EXAMPLE 2

### Composition of myrrh/pelargonium/mannitol/sucresters in a ratio w/w 15/15/50/20

For the preparation of the composition of this example, 15 g of a mixture in the ratio w/w 15/15/50/20 of myrrh (2.25 g)/pelargonium (2.25 g)/mannitol (7.5 g)/sucresters (3 g), obtained using a rotating body mixer, were loaded into a jar of a planetary mill and subjected to co-grinding for 40 minutes at a speed of 200 rpm. At the end of the process, the product in a fine powder form was unloaded and sieved. A co-ground myrrh/pelargonium/mannitol/sucresters in a ratio w/w 15/15/50/20 was obtained titrated in myrrh at 15% by weight and in pelargonium at 15% by weight.

### EXAMPLE 3

### Composition myrrh/boswellia/mannitol/sucresters/arginine in a ratio w/w 20/15/52/10/3

For the preparation of the composition of this example, 15 g of a mixture in the ratio w/w 20/15/52/10/3 of myrrh (3 g)/boswellia (2.25 g)/mannitol (7.8 g)/sucresters (1.5 g)/arginine 0.45 g), obtained using a rotating body mixer, were loaded into a jar of a planetary mill and subjected to co-grinding for 30 minutes at a speed of 200 rpm. At the end of the process, the product in a fine powder form was unloaded and sieved. A co-ground myrrh/boswellia/mannitol/sucresters/arginine in a ratio w/w 20/15/52/10/3 was obtained titrated in myrrh at 20% by weight and in boswellia at 15% by weight.

### EXAMPLE 4

### Composition myrrh/boswellia/fructose/sucresters/arginine in a ratio w/w 10/20/60/7/3

For the preparation of the composition of this example, 30 g of a mixture in the ratio w/w 10/20/60/7/3 of myrrh (3 g)/boswellia (6 g)/fructose (18 g)/sucresters (2,1 g)/arginine 0,9 g), obtained using a rotating body mixer, were loaded into a jar of a planetary mill and subjected to co-grinding for 45 minutes at a speed of 150 rpm. At the end of the process, the product in a fine powder form was unloaded and sieved. A co-ground myrrh/boswellia/fructose/sucresters/arginine in a ratio w/w 10/20/60/7/3 was obtained titrated in myrrh at 10% by weight and in boswellia at 20% by weight.

### EXAMPLE 5

### Composition of myrrh/propolis/mannitol/sucresters in a ratio w/w 20/15/50/15

For the preparation of the composition of this example, 2 kg of a mixture in the ratio w/w 20/15/50/15 of myrrh (400 g)/propolis (300 g)/mannitol (1,000 g)/sucresters (300 g), obtained using a rotating body mixer, were loaded into a vibrational mill suitably adjusted to obtain an oscillation amplitude between 6 and 10 mm and subjected to co-grinding for 30 minutes. At the end of the process, the product in a fine powder form was unloaded and sieved. A co-ground myrrh/propolis/mannitol/sucresters in a ratio w/w 20/15/50/15 was obtained titrated in myrrh at 20% by weight and in propolis at 15% by weight.

### EXAMPLE 6

### Composition myrrh/propolis/fructose/sucresters in a ratio w/w 10/20/55/15

For the preparation of the composition of this example, 30 g of a mixture in the ratio w/w 10/20/55/15 of myrrh (3 g)/propolis (6 g)/fructose (16.5 g)/sucresters (4.5 g), obtained using a rotating body mixer, were loaded into a jar of a planetary mill and subjected to co-grinding for 15 minutes at a speed of 300 rpm. At the end of the process, the product in a fine powder form was unloaded and sieved. A co-ground myrrh/propolis/mannitol/sucresters in a ratio w/w 10/20/55/15 was obtained titrated in myrrh at 10% by weight and in propolis at 20% by weight.

### EXAMPLE 7

### Composition myrrh/echinacea/fructose/sucresters in a ratio w/w 10/20/50/20

For the preparation of the composition of this example, 20 kg of a mixture in the ratio w/w 10/20/50/20 of myrrh (2 kg)/echinacea (4 kg)/fructose (10 kg)/sucresters (4 kg), obtained using a drum tilter, were loaded into a vibrational mill suitably adjusted to obtain an oscillation amplitude between 6 and 10 mm and subjected to co-grinding for 25 minutes. At the end of the process, the product in a fine powder form was unloaded and sieved. A co-ground myrrh/echinacea/fructose/sucresters in a ratio w/w 10/20/50/20 was obtained titrated in myrrh at 10% by weight and in echinacea at 20% by weight.

### EXAMPLE 8

### Composition myrrh/echinacea/mannitol/sucresters in a ratio w/w 20/15/45/20

For the preparation of the composition of this example, 30 g of a mixture in the ratio w/w 20/15/45/20 of myrrh (6 g)/echinacea (4.5 g)/mannitol (13.5 g)/sucresters (6 g), obtained using a rotating body mixer, were loaded into a jar of a planetary mill and subjected to co-grinding for 20 minutes at a speed of 250 rpm. At the end of the process, the product in a fine powder form was unloaded and sieved. A co-ground myrrh/echinacea/mannitol/sucresters in a ratio w/w 20/15/45/20 was obtained titrated in myrrh at 20% by weight and in echinacea at 15% by weight.

Below are examples relating to co-ground compositions containing myrrh in combination with fructose or mannitol and sucresters.

### EXAMPLE A

### Composition of myrrh/fructose/sucresters in ratio w/w 12.5/62.5/25

For the preparation of the composition of this example, 30 g of a mixture in the ratio w/w 12.5/62.5/25 of myrrh (3.75 g)/fructose (15.75 g)/sucresters (7.5 g), obtained using a rotating body mixer, were loaded into a jar of a planetary mill and subjected to co-grinding for 20 minutes at a speed of 300 rpm. At the end of the process, the product in a fine powder form was unloaded and sieved. A co-ground myrrh/fructose/ sucresters in a ratio w/w 12.5/62.5/25 was obtained titrated in myrrh at 12.5% by weight.

### EXAMPLE B

### Composition myrrh/mannitol/sucresters in ratio w/w 15/55/30

For the preparation of the composition of this example, 30 g of a mixture in the ratio w/w 15/55/30 of myrrh (4.5 g)/mannitol (16.5 g)/sucresters (9 g), obtained using a rotating body mixer, were loaded into a jar of a planetary mill and subjected to co-grinding for 30 minutes at a speed of 250 rpm. At the end of the process, the product in a fine powder form was unloaded and sieved. A co-ground myrrh/mannitol/ sucresters in a ratio w/w 15/55/30 was obtained titrated in myrrh at 15% by weight.

### EXAMPLE 9

### Determination of solubility in water

The Applicants conducted tests to evaluate the solubility in water at 25 ° C of some compositions of the present invention, exemplified according to Examples 1 - 8 and A and B, with respect to some comparison systems, such as the starting raw materials or the simple physical mixtures of powders. The physical mixtures were obtained by weighing the components in the ratios indicated in the respective examples and mixing them in shaker-rotating mixer.

For example, to obtain the physical mixture MIX FIS M1 corresponding to the co-ground composition of Example 1, the components were weighed in the correct quantities [myrrh (3g), pelargonium (6g), fructose (12g) and sucresters (9g)] in a container chosen taking care that the volume of the powders does not exceed 1/3 of the volume of the container, for example a cylindrical jar in food-grade polythene with a lid; the jar was then placed tightly closed in a stirrer / mixer (for example Turbula^{™} or similar) and operated for the time necessary to achieve visual uniformity of the content, for example 15 minutes.For the analysis of the solubility, an excess of powder sample was placed in water at 25 ° C, in order to have a sediment, and was kept stirred, for example with a magnetic stir bar.

After 1 hour with the suitable analytical method (for example UV spectrophotometry) the quantity of extracts that passes into solution, filtering and optionally diluting the samples, if needed, was verified.

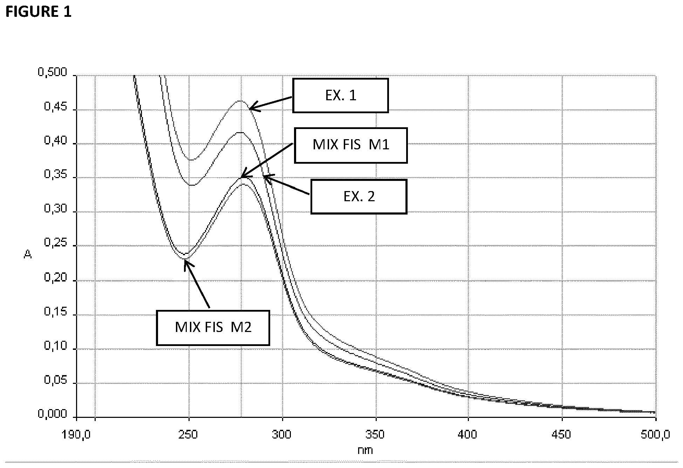

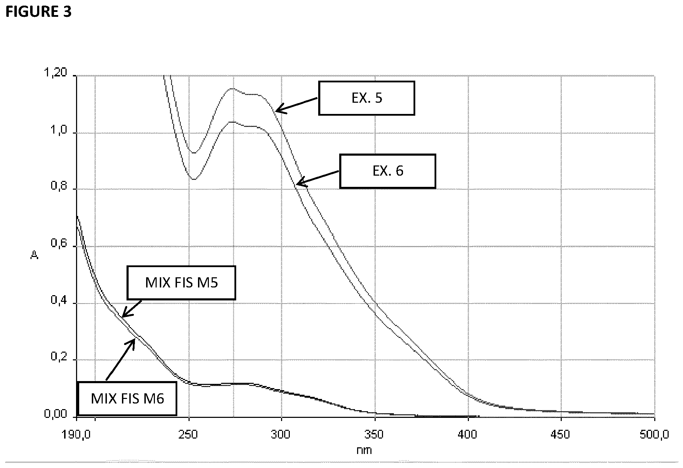

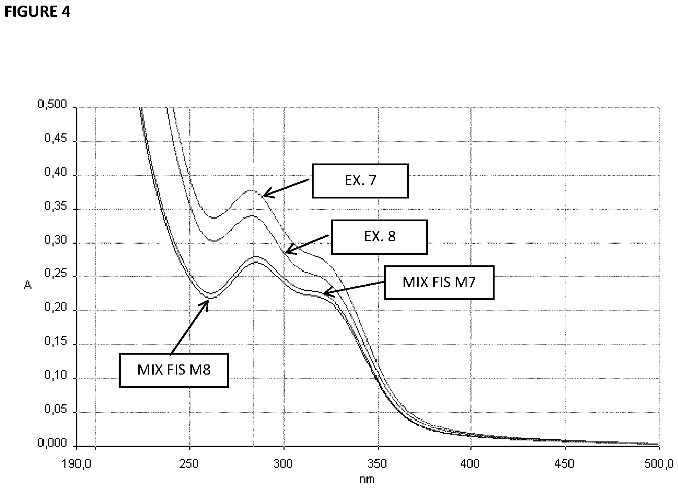

Figures 1 to 4 show the comparison spectra between the solubilities of the physical mixtures and the respective co-ground. In the Figures, the physical mixtures are identified with the initials "MIX FIS" followed by M plus the number of the example of the corresponding co-ground.

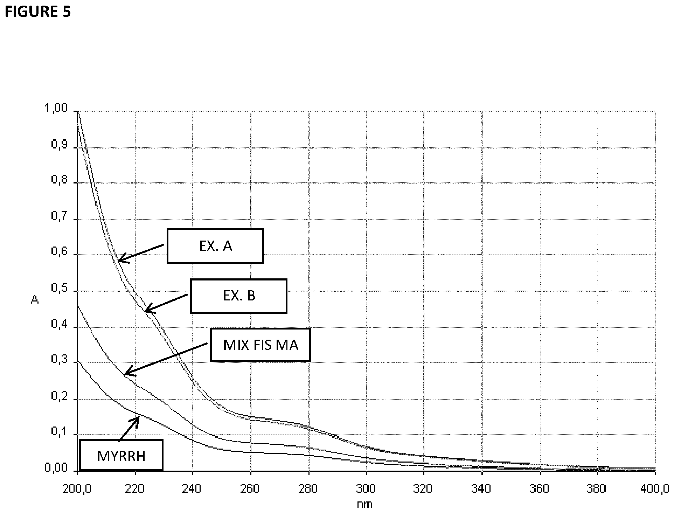

Also shown (Figure 5) are the comparative spectra of the solubility of myrrh with respect to the comacinates with fructose or mannitol and sucresters.

Tables 1 to 5 show the values of the absorbances (Abs) measured at the wavelength corresponding to the maximum peak. The upward arrow indicates the percentage increase in solubility of the co-ground with respect to the corresponding physical mixture.

TABLE 1 shows the absorbance values (Abs) at the wavelength of 278 nm and the percentage increases in solubility of the co-ground of Example 1 and 2 with respect to the corresponding physical mixtures of the components M1 and M2.

**TABLE 1**

| | Abs | ↑ |
|---|---|---|
| MIX FIS M1 | 0.35 | |
| EXAMPLE 1 | 0.46 | 31% |
| MIX FIS M2 | 0.33 | |
| EXAMPLE 2 | 0.42 | 27% |

TABLE 2A shows the Absorbance (Abs) values read at the wavelength of 292 nm and the percentage increases in solubility of the co-ground products of Example 3 and 4 with respect to the corresponding physical mixtures of the M3 and M4 components; TABLE 2B shows for comparison the values of the derivatives of the absorbances (δAbs) measured at the same wavelength on the spectrum shown in FIG. 2B - the results of the percentage increase in solubility are in agreement, demonstrating the validity of the method adopted.

**TABLE 2A**

| | δAbs | ↑ |
|---|---|---|
| MIX FIS M3 | 0.04 | |
| EXAMPLE 3 | 0.24 | 590% |
| MIX FIS M4 | 0.02 | |
| EXAMPLE 4 | 0.18 | 800% |

**TABLE 2B**

| | Abs | ↑ |
|---|---|---|
| MIX FIS M3 | 0.14 | |
| EXAMPLE 3 | 0.99 | 610% |
| MIX FIS M4 | 0.08 | |
| EXAMPLE 4 | 0.71 | 790% |

TABLE 3 shows the values of the Absorbances (Abs) at the wavelength of 274 nm and the percentage increases in solubility of the co-ground of Example 5 and 6 with respect to the corresponding physical mixtures of the components M5 and M6

**TABLE 3**

| | Abs | ↑ |
|---|---|---|
| MIX FIS M5 | 0.12 | |
| EXAMPLE 5 | 1.15 | 860% |
| MIX FIS M6 | 0.11 | |
| EXAMPLE 6 | 1.04 | 850% |

TABLE 4 shows the absorbance values (Abs) at the wavelength of 284 nm and the percentage solubility increases of the co-ground products of Example 7 and 8 with respect to the corresponding physical mixtures of the components M7 and M8.

**TABLE 4**

| | Abs | ↑ |
|---|---|---|
| MIX FIS M7 | 0.28 | |
| EXAMPLE 7 | 0.38 | 40% |
| MIX FIS M8 | 0.27 | |
| EXAMPLE 8 | 0.34 | 30% |

TABLE 5A shows the values of the Absorbances (Abs) at the wavelength of 277 nm and the percentage increases in solubility of the co-ground products of Example A and B with respect to myrrh.

Table 5B shows the absorbance values (Abs) at the wavelength of 277 nm and the percentage increases in solubility of the co-ground of Example A with respect to the physical mixture of its components (MA).

**TABLE 5A**

| | Abs | ↑ |
|---|---|---|
| MYRRH | 0.04 | |
| EXAMPLE A | 0.13 | 230% |
| EXAMPLE B | 0.12 | 210% |

**TABELLA 5B**

| | Abs | ↑ |
|---|---|---|
| MIX FIS MA | 0.06 | |
| EXAMPLE A | 0.13 | 120% |

## Claims

1. A composition comprising a dry extract of *Commiphora myrrho,* fructose or mannitol and sucrose esters, optionally with aminoacids, obtained by dry co-grinding of its components, optionally comprising a further ingredient, selected from the group comprising dry extract of *Boswellia serrata, Pelargonium sidoides, Echinacea purpurea* and propolis, provided that said composition obtained by co-grinding does not contain polymeric excipients.

2. The composition according to claim 1, wherein each dry extract is present in a ratio between 5 and 50% by weight, optionally between 10 and 30% by weight.

3. The composition according to claim 1, wherein fructose or mannitol are present in the composition in a ratio between 20 and 80% by weight, optional between 40 and 60% by weight.

4. The composition according to claim 1, wherein the sucrose esters, optionally with aminoacids, are present in the composition in a ratio between 5 and 40% by weight, optionally between 10 and 30% by weight.

5. The composition according to claim 1, comprising a dry extract of *Commiphora myrrho,* a dry extract of *Pelargonium sidoides,* fructose and sucrose esters, obtained by dry co-grinding of its components.

6. The composition according to claim 1, comprising a dry extract of *Commiphora myrrho,* dry extract of *Boswellia serrata,* mannitol, sucrose esters and arginine, obtained by dry co-grinding of its components.

7. The composition according to claim 1, comprising a dry extract of *Commiphora myrrho,* a dry extract of *Echinacea purpurea,* fructose and sucrose esters, obtained by dry co-grinding of its components.

8. The composition according to claim 1, comprising a dry extract of *Commiphora myrrho,* propolis, mannitol and sucrose esters, obtained by dry co-grinding of its components.

9. A food supplement comprising the composition according to any one of claims 1 to 8.

10. A medical device comprising the composition according to any one of claims 1 to 8.

11. A food for special medical purposes comprising the composition according to any one of claims 1 to 8.

## Patentansprüche

1. Zusammensetzung, umfassend einen Trockenextrakt von *Commiphora myrrha,* Fructose oder Mannitol und Saccharoseester, optional mit Aminosäuren, die durch trockenes Zusammenvermahlen ihrer Bestandteile erhalten wird, optional umfassend einen weiteren Bestandteil, der aus der Gruppe ausgewählt ist, die Trockenextrakt von *Boswellia serrata, Pelargonium sidoides, Echinacea purpurea* und *Propolis* umfasst, mit der Maßgabe, dass die durch Zusammenvermahlen erhaltene Zusammensetzung keine polymeren Hilfsstoffe enthält.

2. Zusammensetzung nach Anspruch 1, wobei jeder Trockenextrakt in einem Verhältnis zwischen 5 und 50 Gew.-%, optional zwischen 10 und 30 Gew.-%, vorhanden ist.

3. Zusammensetzung nach Anspruch 1, wobei Fructose oder Mannitol in der Zusammensetzung in einem Verhältnis zwischen 20 und 80 Gew.-%, optional zwischen 40 und 60 Gew.-%, vorhanden sind.

4. Zusammensetzung nach Anspruch 1, wobei die Saccharoseester, optional mit Aminosäuren, in der Zusammensetzung in einem Verhältnis zwischen 5 und 40 Gew.-%, optional zwischen 10 und 30 Gew.-%, enthalten sind.

5. Zusammensetzung nach Anspruch 1, umfassend einen Trockenextrakt von *Commiphora myrrha,* einen Trockenextrakt von *Pelargonium sidoides,* Fructose und Saccharoseester, die durch trockenes Zusammenvermahlen ihrer Bestandteile erhalten wird.

6. Zusammensetzung nach Anspruch 1, umfassend einen Trockenextrakt aus *Commiphora myrrha,* einen Trockenextrakt aus *Boswellia serrata,* Mannitol, Saccharoseester und Arginin, die durch trockenes Zusammenvermahlen ihrer Bestandteile erhalten wird.

7. Zusammensetzung nach Anspruch 1, umfassend einen Trockenextrakt aus *Commiphora myrrha,* einen Trockenextrakt aus *Echinacea purpurea,* Fructose und Saccharoseester, die durch trockenes Zusammenvermahlen ihrer Bestandteile erhalten wird.

8. Zusammensetzung nach Anspruch 1, umfassend einen Trockenextrakt aus *Commiphora myrrha,* Propolis, Mannitol und Saccharoseester, die durch trockenes Zusammenvermahlen ihrer Bestandteile erhalten wird.

9. Nahrungsergänzungsmittel, umfassend die Zusammensetzung nach einem der Ansprüche 1 bis 8.

10. Medizinische Vorrichtung, umfassend die Zusammensetzung nach einem der Ansprüche 1 bis 8.

11. Lebensmittel für besondere medizinische Zwecke, umfassend die Zusammensetzung nach einem der Ansprüche 1 bis 8.

## Revendications

1. Composition comprenant un extrait sec de *Commiphora myrrha,* du fructose ou du mannitol et des esters de saccharose, en option avec des acides aminés, obtenue par co-broyage à sec de ses composants, en option comprenant un ingrédient supplémentaire, choisi dans le groupe comprenant l'extrait sec de *Boswellia serrata, Pelargonium sidoides, Echinacea purpurea* et propolis, étant entendu que ladite composition obtenue par co-broyage ne contient pas d'excipients polymères.

2. Composition selon la revendication 1, dans laquelle chaque extrait sec est présent en un rapport compris entre 5 et 50 % en poids, en option entre 10 et 30 % en poids

3. Composition selon la revendication 1, dans laquelle le fructose ou le mannitol est présent dans la composition en un rapport compris entre 20 et 80 % en poids, en option entre 40 et 60 % en poids.

4. Composition selon la revendication 1, dans laquelle les esters de saccharose, en option avec des acides aminés, sont présents dans la composition en un rapport compris entre 5 et 40 % en poids, en option entre 10 et 30 % en poids.

5. Composition selon la revendication 1, comprenant un extrait sec de *Commiphora myrrha,* un extrait sec de *Pelargonium sidoides,* du fructose et des esters de saccharose, obtenue par co-broyage à sec de ses composants.

6. Composition selon la revendication 1, comprenant un extrait sec de *Commiphora myrrha,* un extrait sec de *Boswellia serrata,* du mannitol, des esters de saccharose et de l'arginine, obtenue par co-broyage à sec de ses composants.

7. Composition selon la revendication 1, comprenant un extrait sec de *Commiphora myrrha,* un extrait sec *d'Echinacea purpurea,* du fructose et des esters de saccharose, obtenue par co-broyage à sec de ses composants.

8. Composition selon la revendication 1, comprenant un extrait sec de *Commiphora myrrha,* de la propolis, du mannitol et des esters de saccharose, obtenue par obtenue par co-broyage à sec de ses composants.

9. Complément alimentaire comprenant la composition selon l'une quelconque des revendications 1 à 8.

10. Dispositif médical comprenant la composition selon l'une quelconque des revendications 1 à 8.

11. Aliment à fins médicales particulières comprenant la composition selon l'une quelconque des revendications 1 à 8.
